# EUROPEAN PATENT APPLICATION

(11) **EP 4 495 131 A1**
(43) Date of publication of application: **22.01.2025**
(21) Application number: 23306262.9
(22) Date of filing: 21.07.2023
(51) Int. Cl.: C07K 14/47, A23C 20/02, A23J 1/00, C12N 1/06, C12R 1/19

(54) **METHOD FOR PRODUCING CASEIN AND USES THEREOF**

(71) Applicant: Standing Ovation, 75014 Paris (FR)
(72) Inventor: Chayot, Romain, 75014 Paris (FR); Lorilliere, Marion, 91280 Saint Pierre Du Perray (FR)
(74) Representative: Flesselles, Bruno F.G.

(57) **Abstract**

The invention relates to new methods for producing casein compositions by increasing casein ratio in compositions through heating of the composition for a short time through a pipe, and uses thereof, in particular for producing cheese substitute.

## Description

The invention pertains to the food industry and relates to new methods for producing casein compositions and uses thereof, in particular for producing cheese substitute (notably vegan cheese).

### Background

The use of milk as a protein, sugar and lipid-rich nutrient, has become almost universal in traditional societies. In addition, milk transformation into various derivatives, is one of the most ancient examples of human agro-industries. Today, the cheese market represents 20 million tons of product per year, for a value of about $140bn.

However, dairy products, are associated with several issues or concerns, in terms of health, as well as environmental and ethical considerations. These concerns have highlighted the need for dairy product substitutes alleviating these various issues. Health issues include lactose intolerances, allergies (Mousan and Kamat (2016) Cow's Milk Protein Allergy. Clin Pediatr (Phila), Volume 55(11) pages 1054-63.; Manuyakorn and Tanpowpong (2018) Cow milk protein allergy and other common food allergies and intolerances. Paediatr Int Child Health, Volume 39(1), pages 32-40), and others, such as a high content in saturated fatty acids, known to have a potential negative impact on health. (Micha and Mozaffarian (2010) Saturated fat and cardiometabolic risk factors, coronary heart disease, stroke, and diabetes: A fresh look at the evidence. Lipids, Volume 45, pages 893-905; Jakobsen et al (2009) Major types of dietary fat and risk of coronary heart disease: A pooled analysis of 11 cohort studies. Am. J. Clin. Nutr; Volume 89, pages 1425-1432; Nettleton et al (2017) Saturated fat consumption and risk of coronary heart disease and ischemic stroke: A science update. Ann Nutr Metab Volume 70, pages 26-33). Lactose intolerance is due to the lack of lactase, the enzyme that degrades lactose in the stomach and small intestine, and result in an accumulation of lactose in the colon, and its digestion by bacteria (Ugidos-Rodriguez et al (2018) Lactose malabsorption and intolerance: a review. Food Funct, Volume 9(8), pages 4056-4068). It is a very common feature in humans, is genetically determined, and results in the need for a dairy-free diet. In a dairy product substitute, this composition could be modulated, to avoid lactose, and saturated fatty acids, and even, allergenic proteins.

In addition, environmental and ethical concerns related to food products of animal origin have raised over the last decades. The burden of animal breeding for a 7 billion population (11 billion in 2050) has become increasingly high. The environmental consequences are severe. They are today mostly considered in terms of anthropogenic greenhouse gas (GHG) emissions, water consumption, pollution by effluents, and land occupation.

Cattle breeding is today considered as one of the first sources GHG emissions, with an estimated 7.1 Gigatonnes of CO2-equivalent per year, representing 14,5% of all anthropogenic greenhouse gas (GHG) emissions the sources and methods (Rotz (2017) Modeling greenhouse gas emissions from dairy farms. J Dairy Science, Volume 101, pages 6675-6690), but a study from the UN estimated in 2010 the contribution of the global dairy sector alone to 4% of anthropogenic GGHG emissions (FOOD AND AGRICULTURE ORGANIZATION OF THE UNITED NATIONs, Greenhouse Gas Emissions from the Dairy Sector. A Life Cycle Assessment).

A very strong water consumption is also associated with animal farming (Sultana et al (2014). Comparison of water use in global milk production for different typical farms. Agricultural Systems, Volume 129, pages 9-21; Ercin and Aldaya (2012) The water footprint of soy milk and soy burger and equivalent animal products. Ecological Indicators, Volume 18, pages 392-402). In addition, farming effluents also have a strong environmental impact. Although the impact of crop fertilizers on groundwater should not be neglected, the impact of animal farming effluents is often massive and has proven to be disastrous in many parts of the world (https://www.nrdc.org/issues/livestock-production). Furthermore, livestock takes up to nearly 80% of global agricultural land, yet produces less than 20% of the world's supply of calories, showing the very high level of stress brought by animal farming on land resources.

Finally, animal welfare has become an increasingly important concern. The scale-up of meat and dairy production and processing has turned it into an intensive industrial process, which is increasingly perceived as ethically not acceptable.

Therefore, there is a strong need for dairy products substitutes alleviating the issues listed above.

Plant-based substitutes are a potential alternative to conventional dairy products. However, these products, which are often derived from starch, soy, almond, or coconut milk, can be far from mimicking the taste of dairy products. In addition, they are anyway fundamentally different in terms of composition.

Therefore, there is today a need for dairy substitutes, and in particular, cheese substitutes
(i) free of undesired compounds
(ii) similar to the original product in aspect, texture and taste
(iii) equivalent or superior to the original products in terms of nutrition

Cows' milk typical composition is described in Table 1 and more detailed compositions of cow's and other animals milk, including the listing of the different lipids, proteins, salts, vitamins and other nutrients can be found from a large number of sources (https://en.wikipedia.org/wiki/Milk#Cow's_milk; Haug et al (2007) Bovine milk in human nutrition - a review, Lipids Health Dis.; Volume 6, pages 25; Dominguez-Salasa et al (2019) Contributions of Milk Production to Food and Nutrition Security; Encyclopedia of Food Security and Sustainability, Volume 3, pages 278-291).

**Table 1: Cow's milk composition**

| Type of molecule | mass in 1 liter in grams |
|---|---|
| Water | 902 |
| Lactose | 49 |
| Fat | 39 |
| Caseins | 28 |
| Other proteins | 5 |
| Salts and vitamins | 9 |
| Total | 1032 |

Lipids and carbohydrates (but lactose) can be recovered from plant, calcium from inorganic sources, from animal sources or from plants (such as sea weeds). In the case of proteins, other sources have to be considered for animal proteins differ in amino-acid content from plant proteins. Milk's proteins can also be produced by fermentation, another source of ingredients of a non-animal origin. Furthermore, proteins isolated form milk are also individually used as nutritional supplements and other applications. Therefore, milk proteins made by fermentation, independently of other milk components can also be used for other applications than the making of dairy substitutes.

Production by fermentation is based on the growth of bacteria or fungi producing a compound of interest in a fermenter, usually followed by the recovery and purification of the compound of interest. The making of proteins by fermentation is a process that has widely been used in the food industry. Several studies have described the production of milk's constitutive proteins or homologues by fermentation in various microorganisms (see below) and the assembly of ingredients including fermentation proteins to make dairy substitutes has been described several times (US6270827; US5,942,274; WO2018039632; WO2020223700; WO2020081789, WO2020219596, WO2022098835).

Production by fermentation us usually conducted with a sugar feedstock, to support the growth of the microorganism and the production of the desired compound. Depending on the capacity of the microbe to metabolize the different sugars, one can use glucose or sucrose or other sugars such as lactose.

For example Escherichia coli is known to consume glucose very efficiently, but can also consume a series of other sugars, including glucose, maltose, lactose, trehalose, fructose, xylose and arabinose with a hierarchy of preference (Luo et al. (2014) The transport and mediation mechanisms of the common sugars in Escherichia coli, Biotechnology Advances volume 32, pages 905-919; Postma et al. (1993) Phosphoenolpyruvate:Carbohydrate Phosphotransferase Systems of Bacteria, Microbiolgical Reviews Sept 1993, pages 543-594). Sucrose can also be metabolized by specific Escherichia coli strains, expressing specific genes corresponding to the Scr or Csc systems (Bruschi et al. (2012) A transferable sucrose utilization approach for non-sucrose-utilizing Escherichia coli strains. Biotechnology Advances 30 (2012) 1001-1010.Sugar-rich feedstock can present various level of purities, such as glucose syrups, sucrose syrups, molasses and vinasses (Suhaili et al. (2018) Potential of sugar beet vinasse as a feedstock . J Chem Technol Biotechnol volume 94, pages 739-751; Naspolini et al. (2017, Bioconversion of Sugarcane Vinasse into High-Added Value Products and Energy, BioMed Research International, vol. 2017, Article ID 8986165, 11 pages) and other products of sugar beet processing (Tomaszewskaet al. (2018) Products of sugar beet processing as raw materials for chemicals and biodegradable polymers. Royal Society of Chemistry Advances, volume 8, pages 3161-3177).

Microorganism growth and metabolism requires not only sugar feedstock, nut also nitrogen input. For this, various compounds can be added, including liquid or gaseous ammonia, urea, amino acids, peptones, and yeast extract.

Alternative feedstocks derived from dairy, such as lactoserum (whey) have also been used in fermentation with various microorganisms comprising Escherichia coli (Carranza-Saavedra (2021) Kinetic analysis and modeling of L-valine production in fermentation batch from E. coli using glucose, lactose and whey as carbon sources; Biotechnology Reports 31 (2021) e00642; Chaparro et al. (2021) Whey as an Alternative Nutrient Medium for Growth of Sporosarcina pasteurii and its Effect on CaCO3 Polymorphism and Fly Ash Bioconsolidation. Materials volume 14, 2470; Christensen et al. (2011) Production of bioethanol from organic whey using Kluyveromyces marxianus J Ind Microbiol Biotechnol volume 38, pages 283-289; Hausjell et al. Valorisation of cheese whey as substrate and inducer for recombinant protein production in E. coli HMS174(DE3) Bioresource Technology Reports 8 (2019) 100340; Loasté and Elourtassi (2020) Succinic acid production from whey and lactose by Actinobacillus succinogenes 130Z in batch fermentation; Biotechnology Reports 27 (2020) e00481; Zou and Chang (2022) Past, Present, and Future Perspectives on Whey as a Promising Feedstock for Bioethanol Production by Yeast; Journal of Fungi, volume 8, page 395 ).

Lactoserum, is rich in lactose and in other nutrients, and intense investigations have been conducted to valorize whey, and notably acid whey (Rocha-Mendoza et al (2021) Invited review: Acid whey trends and health benefits; J. Dairy Sci. volume 104, pages 1262-1275). Using this type of feedstock does not qualify for the making of animal-free products, given the origin of whey. Nevertheless, the use of a waste as feedstock can be very helpful to reach good economics and advantageous life cycle analysis.

During fed batch fermentation, pH can be adapted to keep within a certain range, and for this addition of acidic or basic compounds can be used, including hydrochloric acid, sulfuric acid, lactic acid, and phosphoric acid (acidic compounds) or liquid or gaseous ammonia, sodium hydroxyde, or potassium acid (basic compounds).

All these solutions are not exclusive and can be combined.

However, the industrial production of milk's protein also requires adapted purification procedures, that not only produce the required proteins at a grade compatible with use in the food industry, with a high level of purity, but are also easily scalable at industrial level, and at costs compatible with exploitation, in particular energy costs. Ideally, such procedures should be as simple as possible, and avoid the use of non-edible chemicals and of steps that are expensive when conducted at very high scale.

In the case of recombinant proteins made by fermentation, WO2022253816 describes a procedure to isolate the recombinant casein protein from the biomass and from the culture broth, by heating the composition. In this application, the microorganism composition is heated in a container at a temperature that is between 75°C and 105°. The examples show that the duration of heat is at least 10 minutes, and rather at least 30 minutes or even 120 minutes. It is advised to heat the composition for at least one hour.

The conditions proposed in WO2022253816 require energy to overcome the inertia of the still composition, and the duration of heating is long. The Applicant has shown that it is possible to reduce the duration of heat and maintain a good yield of casein recovery by performing heating for a few minutes at a high temperature, while circulating the composition containing the caseins and other proteins from one container to another container. Heating is performed during the transfer of the casein from the first container to the second container. It is also foreseen that further purification steps (such as centrifugation and/or filtration) are present after the heating step before the composition is delivered to the second container. This method makes it possible to decrease the duration of the casein production process so that it can better be included in an industrial process. This also allows energy saving as heating the pipe in which the composition moves consumes less energy than heating the container in which the fermentation process was performed.

In the context of the present invention, a "casein" is any casein protein or mixture of casein protein. Thus, a "casein" is an alpha-S1 casein, an alpha-S2 casein, a beta casein, or a kappa casein. In some extend, it can also designate any mixture of such proteins. One can use the term "casein" or "caseins" to discuss casein proteins in general.

In the context of the invention, the term "between" includes the limits.

In the context of the present invention, the term "cheese substitute" means a food product having the essential features of cheese in terms of nutritional value, aspect, texture and taste.

The term "fresh cheese" designates a cheese with a moisture higher than 80% on a fat-free basis (ratio of water vs. total mass of product without fat), and a protein content comprised between 2% and 15% of total weight (ratio of protein mass vs. total product mass).

The term "soft cheese" or "semi-soft cheese" designates a cheese with a moisture comprised between 62% and 80% on a fat-free basis (ratio of water vs. total mass of product without fat), and a protein content comprised between 15% and 30% of total weight (ratio of protein mass vs. total product mass). A soft cheese has a moisture comprised between 67% and 80%, on a fat-free basis, and a semi-soft cheese has a moisture comprised between 62% and 67%, on a fat-free basis.

In the context of the present invention, the term "liquid pre-curd composition" or "LpCC" designates the composition containing at least a casein and at least one other ingredient comprising at least a component among water, calcium, lipids, an carbohydrate, before the addition of rennet and ferments and curdling (in particular coagulation of casein). The casein concentration in the LpCC is higher than that in milk. The concentration of the other ingredient in the LpCC is also higher than that in milk. This makes it possible to save some water when using a LpCC with recombinant caseins as disclosed in the present application, as the LpCC doesn't represent an artificial (cow's) milk. The respective concentrations of proteins, lipids, calcium salts and/or carbohydrates are adjusted to fit the final desired compositions of cheese substitutes

In the context of the present invention, the term "curdling agent" designates a chemical or biochemical composition capable of triggering curdling. Curdling can be achieved by the addition of acid solutions, by the addition of starter cultures (ferments which growth in the presence of carbohydrates triggers a decrease of pH), by heat treatment, by addition of calcium chelating agent, by the addition of natural or recombinant rennet, by the addition of rennet substitutes, such as animal proteases, or vegetal curdling enzymes), or by a combination of these processes. A curdling agent may be any additive, compound, composition or treatment which addition results in curdling, alone or in combination with another or others additives, compounds, compositions or treatments. When preparing animal-free edible compositions, it is preferred to use an acidifying agent (such as an acid or starter cultures), in order to avoid using rennet, so that the curdling agent does not comprise any element of animal origin. It is advantageous when the acidifying agent comprises a lactic bacterium or lactic bacteria, but acidic chemicals can also be used.

In the context of the present invention, the term "curd" means a composition wherein casein coagulates or precipitate, under the action of the curdling agent, and which can be separated from a liquid phase, if any, by draining, for example on a cheese cloth. The curd also comprises other ingredients, including water and lipids (when present),

In the context of the present invention, the term "ferment" designates a composition containing at least one microbial strain, added during the process of production of the cheese substitute. Lactic ferments are responsible for lactic fermentation, resulting notably in an acidification of the medium. As a consequence, they can be used as a curdling agent.

Other ferments are used in cheese production for the processing of the curd, resulting in modifications of texture, taste, smell, and chemical composition (with notably the cleavage of proteins into smaller peptides). These ferments can be called "ferments for maturation", "maturation ferments" or "ripening ferments" and are generally not used for production of fresh cheeses. Such ferments for maturation can be added together with the curdling agent.

In the context of the present invention, the term "of non-animal origin" means a compound or composition which has not been directly derived from an animal, produced from animal cells in culture, or isolated from animal products such as milk. Compounds or compositions produced by fermentation of microbes are thus "of non-animal origin" even though some products of animal origin, bacto peptone for example, can be involved during fermentation. Therefore, in the context of the invention, a protein that is naturally produced in animals will be called of non-animal origin when it is produced in microbial (such as bacterial or yeast) cells or in plant cells, even though its sequence or structure may be identical to the sequence or structure of the protein that would be isolated from animal.

In the context of the present invention, the term "animal-free" means a compound or composition which has not been derived from an animal, from animal cells in culture, or from animal products such as milk, and whose production process does not involve any feedstock or additive of animal origin.

In the context of the invention, the term "texturing agent" means any gelling agent, including emulsifier such as lecithin, and hydrocolloids such as cassia gum, sesbania gum, tamarind gum, guar gum, fenugreek gum, Arabic gum, agar agar (or agar-agar), carrageenans, tragacanth gum, xanthan gum, carob (locust bean) gum, cellulose gum.

In a first aspect, is herein disclosed a method for the solubilization of casein from a composition comprising a soluble and an insoluble fraction containing casein and other proteins, wherein casein is predominantly present in the insoluble fraction of the composition, comprising:
i) providing, in a first container, a composition comprising a soluble and an insoluble fraction containing casein and other proteins, wherein casein is predominantly present in the insoluble fraction of the composition, wherein the pH of the composition is equal or above 6.5 and preferably below 9
ii) flowing the composition out of the first container within device comprising a pipe and heating the composition during flowing within the pipe, wherein heating is performed at a temperature equal or above 95°C for at most 5 minutes
iii) recovering the soluble fraction of the flown composition,
wherein casein is enriched in the recovered fraction. Part of the casein which was predominantly present in the non-soluble was thus solubilized. In preferred embodiment, the casein is mainly present in the soluble fraction after performance of the method (thus the soluble fraction is enriched in casein).

The composition is a liquid solution that contains casein and other proteins. It also presents a soluble fraction and a non-soluble fraction. In a specific embodiment, the composition also contains transgenic microorganisms such as bacteria that produce caseins as a result of the presence, in their genome of transgene(s) coding for casein. Such bacteria (whether lyzed or not) are present in the non-soluble fraction.

The composition containing the transgenic microorganism can be obtained by fermentation, using various sugars and nitrogen sources as feedstocks. In a preferred embodiment, fermentation is conducted in a medium containing glucose, sucrose or lactose. In a preferred embodiment, fermentation is conducted in a medium containing glucose, sucrose or lactose as a carbon source. In a preferred embodiment, fermentation is conducted in a medium containing molasses or vinasses as a carbon source. In a preferred embodiment, fermentation is conducted in a medium containing molasses as a carbon source. In a preferred embodiment, liquid or gaseous ammonia, urea, amino acids, peptones, or yeast extract are present or added in the medium as a nitrogen source. In a preferred embodiment, fermentation is conducted on a medium containing ammonia, urea, amino acids, peptones, or yeast extract as a nitrogen source. In a preferred embodiment, fermentation is conducted on a medium containing lactoserum as a feedstock. In a preferred embodiment, fermentation is conducted on a medium containing acid lactoserum as a feedstock. In a preferred embodiment, fermentation is conducted on a medium containing lactoserum and another sugar as additional sugar source. In a preferred embodiment, fermentation is conducted on a medium containing acid lactoserum and glucose, sucrose or lactose as additional sugar source.

Before heating, pH can be adjusted by the addition of as liquid or gaseous ammonia, sodium hydroxide, potassium hydroxide or calcium hydroxide or other basic compounds. pH is superior to 6,5, and preferably inferior to 9.

The composition containing casein and other proteins (as well as potentially microorganisms) is thus flown from the first container via a device containing a pipe or a tube, such tubular exchanger having the functions of both displacing the composition and heating it. There is thus a heat transfer (or heat exchange) during the passing of the composition within the pipe, so as to heat the composition, such transfer being performed on at least a fraction of the pipe.

The soluble fraction may be recovered by performing a centrifugation of the liquid composition flowing from the pipe.

The concentration of casein, and/or the ration of casein versus other proteins is higher in the recovered soluble fraction than in the starting composition, the casein thereby being enriched in this soluble recovered fraction.

As indicated, the composition of i) contains a soluble fraction and an insoluble fraction. The soluble fraction refers to a fraction that is not pelleted upon centrifugation. The insoluble fraction is the pellet obtained after centrifugation. Centrifugation may be performed at about 3000 g for 20-30 minutes. At industrial scale, continuous flow centrifugation, or another method such as filtration may be used to recover the soluble fraction. In the case of filtration, membranes of different types can be used.

The desired technical effect is obtained by heating during the passing in the tube, and preferably in the absence of organic solvents, or of addition thereof.

The device may contain, before the heating pipe zone, a zone (such as a chamber or a length of a pipe) for pre-heating the composition before flowing within the pipe at the desired temperature. For instance, when the composition is at room temperature, the pre-heating may raise the composition temperature at about 80°C. The device may also present, after the heating pipe zone, a zone for cooling the composition, at temperature below 100°C (preferably about 60-80°C at most) for being able to recover the soluble fraction.

The ratio of casein is increased in the soluble fraction, when the relative amount of casein increases as compared to the total amount of proteins.

The inventors have shown that heating the composition when it passes through the pipe thus for a quite short period of time (less than 5 minutes, preferably less than 2 minutes, more preferably less than 1 minute) also makes it possible to increase or maintain the amount of casein in the soluble fraction while reducing the amount of the other proteins in the soluble fraction of the composition, while. Thus, the recovered fraction is enriched in casein.

The other proteins are non-casein proteins. This method thus makes it possible to obtain a composition enriched in casein. Enrichment of casein in the composition refers to an increase of casein in the soluble fraction as compared to the amount present in the soluble fraction of the starting composition before flow through the pipe.

Such method can be used for the isolation or purification of casein from the composition containing casein and other proteins. Such method can also be used for enriching casein in a protein composition, *i.e.* increasing the proportion of casein in the protein fraction of the protein composition.

In the context of the methods herein described, a "casein composition" designates a composition which contains casein. In order to use a purification step based on the separation of the soluble and insoluble fraction, a casein composition needs to comprise a soluble fraction. However, this casein composition can then be desiccated in further steps. In some embodiments, casein represents more than 25 % of the proteins of the composition. In another embodiment casein represents more than 50 % of the proteins of the composition. In come embodiments, casein represents the major part (more than 50%) of dry weight. Such casein composition can also comprise other compounds, notably calcium, other proteins, lipids and others. A casein composition produced by a microbial culture is a casein composition of non-animal origin.

In view of this, it results that the recovered soluble fraction may contain only casein at the end of the process, or may also contain other proteins (when the precipitation and/or degradation of the other proteins upon heating is not complete). However, as indicated above, it is preferred when casein represents at least 50% of the proteins in the recovered soluble fraction (weight/weight) obtained after performing the process. In a preferred embodiment, casein represents at least 60 %, at least 62 %, at least 65 %, at least 67 %, or at least 70%, or at least 71 % or at least 72 %, or at least 80%, or at least 85%, or at least 90%,or at least 91%, or at least 92%, or at least 93%, or at least 94%, or at least 95% of the proteins of the recovered soluble fraction obtained after performing the process. The amount of proteins can be measured by any method known in the art (using a gel, protein dosing).

As indicated above, the composition containing casein and other proteins, which is used at (i) may also contain other components, such as carbohydrate or lipids. This is particularly true when the composition containing casein and other proteins results from a culture of recombinant microorganisms (including eukaryotic cells) expressing casein. Such other components (in totality or part of such) will be present in the casein composition.

Such composition containing casein and other proteins used in (i) may contain the microorganisms suspended in an appropriate buffer or solution but more preferably in water, or the supernatant resulting from the microorganisms' culture, in case the casein is secreted by the microorganisms.

In a specific embodiment, the starting composition (composition containing casein and other proteins) contains alpha-S1 casein. In one embodiment, alpha-S1 casein is the only casein in the starting composition.

In a specific embodiment, the starting composition (composition containing casein and other proteins) contains alpha-S2 casein. In one embodiment, alpha-S2 casein is the only casein in the starting composition.

In a specific embodiment, the starting composition (composition containing casein and other proteins) contains beta casein. In one embodiment, beta casein is the only casein in the starting composition.

In a specific, but non-preferred embodiment, the starting composition (composition containing casein and other proteins) contains kappa casein. Thus in preferred embodiments, the starting composition (composition containing casein and other proteins) doesn't contain kappa casein.

In a specific embodiment, the starting composition (composition containing casein and other proteins) contains a mixture of alpha-S1 and beta caseins. In one embodiment, alpha-S1 and beta caseins are the only caseins in the starting composition.

In a specific embodiment, the starting composition (composition containing casein and other proteins) contains a mixture of alpha-S1 and alpha-S2 caseins. In one embodiment, alpha-S1 and alpha-S2 caseins are the only caseins in the starting composition.

In a specific embodiment, the starting composition (composition containing casein and other proteins) contains a mixture of alpha-S2 and beta caseins. In one embodiment, alpha-S2 and beta caseins are the only caseins in the starting composition.

In a specific embodiment, the starting composition (composition containing casein and other proteins) contains a mixture of alpha-S1, alpha-S2 and beta caseins. In one embodiment, alpha-S1, alpha-S2 and beta caseins are the only caseins in the starting composition.

In a specific embodiment, the starting composition (composition containing casein and other proteins) contains a mixture of alpha-S1, alpha-S2, beta and kappa caseins.

In a specific embodiment, the starting composition (composition containing casein and other proteins) contains a mixture of a combination of two to three caseins chosen among alpha-S1, alpha-S2, beta and kappa caseins.

When the starting composition is obtained after production of casein by a culture of microorganisms, such microorganisms are transgenic for genes allowing production of the casein(s) that it is desired to obtain in the composition.

The soluble fraction may be subjected to another step of purification, such as filtration (in particular ultrafiltration, nanofiltration, reverse osmosis), centrifugation, chromatography, or another precipitation, such as acidic precipitation of casein) to further purify and/or enrich the casein. In particular, acidic precipitation is performed at at a pH between 4 and 5, and preferably at about 90°C. Multiple other steps of purification can be performed.

In a specific embodiment, the microorganisms are bacterial cells. In a preferred embodiment, the microorganisms is Escherichia coli. In another embodiment, the microorganisms are fungi cells (including yeast cells). In another embodiment, the microorganisms are eukaryotic cells, in particular plant cells. It is indeed preferred that the casein is produced in a non-animal organism.

The microorganism can be cultivated by fermentation, using various sugars and nitrogen sources as feedstocks. In a preferred embodiment, fermentation is conducted on a medium containing glucose, sucrose or lactose. In a preferred embodiment, fermentation is conducted on a medium containing glucose, sucrose or lactose as a carbon source. In a preferred embodiment, fermentation is conducted on a medium containing liquid or gaseous ammonia, urea, amino acids, peptones, or yeast extract as nitrogen source. In a preferred embodiment, fermentation is conducted on a medium containing lactoserum. In a preferred embodiment, fermentation is conducted on a medium containing acid lactoserum. In a preferred embodiment, fermentation is conducted on a medium containing acid lactoserum and another sugar as additional sugar source. In a preferred embodiment, fermentation is conducted on a medium containing acid lactoserum and glucose, sucrose or lactose as additional sugar source.

When the casein is produced by way of culture or fermentation of micoorganisms (in particular bacteria) transformed to express casein, one can use the properties of the casein (remain soluble at high temperature, whereas other proteins precipitate and/or are being degraded) to perform a method for the production of a casein composition comprising:
i) providing a microorganism composition (preferably bacterial composition), wherein the microorganism composition comprises microorganisms (preferably bacteria) that have been transformed with at least one nucleic acid coding for a casein, and wherein the microorganisms have been cultured in culture conditions so as to express and produce casein, wherein the pH of the composition is equal or above 6.5 and preferably below 9
ii) optionally washing the microorganism composition to remove the culture medium
iii) optionally lysing the microorganism composition to obtain a liquid composition wherein the liquid composition is in a first container
iv) flowing the liquid composition out of the first container through a device comprising a pipe and wherein the liquid composition is heated at a temperature equal or above 75°C during flowing,
v) recovering the soluble fraction, out of the pipe
thereby obtaining a casein composition.

When this embodiment is performed (casein produced by transgenic microorganisms), casein indeed often remains in the insoluble fractions (such as inclusion bodies in bacteria). Performing the method herein disclose thus makes it possible to solubilize the casein and have it pass from the insoluble to the soluble fraction, thereby providing a liquid soluble casein composition.

The soluble fraction may be recovered by performing a centrifugation of the liquid composition flowing from the pipe, using regular or disc stack centrifuges Alternatively, it can be obtained by microfiltration or diafiltration.

It is possible to optionally perform one further step chosen among addition of activated charcoal, chemical resins, membrane filtration (ultrafiltration, nanofiltration, or reverse osmosis), centrifugation, chromatography or precipitation of casein, to the soluble fraction recovered in v).

In iv), heating the microorganism composition (preferably bacterial composition) shall also induce lysis of the cells, if they are not already lysed, so as to produce a microbial extract, wherein the soluble fraction is enriched in casein and/or wherein the amount of other proteins is reduced in the soluble fraction.

Thus, heating in iv) will enrich in casein in the soluble fraction and/or reduce the amount of the other proteins in soluble fraction, when the microorganism composition comprises already lysed microorganisms, and a further effect of lysis of the microorganisms is observed when the microorganisms present in the microorganism composition are not already lysed. It is indeed foreseen that lysis of the microorganisms may be performed, using any method known in the art, preferably using a chemical (such as detergent) or enzymatic lysis using for instance lysozyme or proteinase K, or mechanically, using a French press.

The enrichment in casein or reduction of other proteins in the soluble fraction is observed as compared to the soluble fraction of the microorganism composition in which the microorganisms have been lysed before heating.

Such method thus allows obtaining a casein composition, and isolation of a casein-rich (or casein-enriched) composition from a microorganism culture. A casein-rich composition is a composition wherein casein represent the major protein. In a preferred embodiment, caseins represent more than 50% of proteins in said casein-rich composition. In a more preferred embodiment, casein represent more than 80%, or 90% or 95% of proteins in said casein-rich composition.

Indeed, and as shown in the examples, upon heating, caseins move from the insoluble fraction (pellet) to the soluble fraction (supernatant). The examples also show that it is essentially casein that is transferred to the soluble fraction upon action of heat, even though duration of heat is less than what is described in WO2022253816.

The microorganism composition contains microorganism cells suspended in an appropriate fluid (such as water or other buffer as explained below). Therefore, it is possible, therefore, upon centrifugation, to obtain an insoluble (pellet) fraction and a soluble fraction (supernatant). It is preferred, however, when the method is performed before centrifugation. In this embodiment, the insoluble fraction can be in suspension within the soluble fraction when heating is performed. Centrifugation, or another method (such as filtration) may thus be used to recover the soluble fraction.

In one embodiment, the microorganism composition (preferably bacterial composition) has been obtained by centrifugation of the microorganism cells (preferably bacteria) after culture, washing, and resuspension in an appropriate liquid or fluid (appropriate buffer and more preferably in water, more preferably in the absence of organic solvents). The buffer is at a pH that is not acidic (equal or above 6.5), preferably close to neutral (between 6.5 and 7.5), or basic (preferably below 9). Indeed, caseins can precipitate at acidic pH, and the process is even enhanced by heating as shown in example 7. It is also preferred when the buffer is not ionic, or with a low ionic strength. The soluble fraction of iii) can be obtained by centrifugation of the composition heated in ii), or by other methods known in the art.

The composition of i) in the methods disclosed above is thus heated when flowing through the pipe, at a temperature comprised higher than 75°C. More preferably, the temperature is higher than about 80°C, more preferably higher than about 85°C, more preferably higher than about 90°C, more preferably higher than about 95°C, most preferably higher than about 100°C, or higher than 100°C. It may be advisable to use temperatures equal or higher than 105°C, or equal or higher than 110°C. It is possible to use temperatures as high as 140°C or even 150°C. It is preferred to heat the composition for 1 minute at most. In this case, an adequate temperature is preferably between 95°C and 115°C, preferably between 100°C and 115°C (limits included). A temperature of about 110°C would be appropriate. Such temperatures make it possible to obtain the technical and functional effects of removal of the other proteins, therefore reducing their amount and/or moving the casein from the insoluble to the soluble fraction, and while heating for a short duration (at most five minutes, preferably at most two minutes, but rather 90 seconds or less, or 75 seconds or less, more preferably 60 seconds or less, or even 45 seconds or less). The examples show that durations of 30 seconds or even 2 seconds can be applied. It is however preferred to heat the composition flowing within the pipe for at least 5 seconds or at least 10 seconds.

Pressure within the pipe is generally higher than 3 bars, more preferably 4 bars or more, more preferably 4.5 bars or more. It is generally 7 bars of less, preferably 6.5 bars or less, preferably 6 bars or less. A pressure between 4.5 and 5.5 bars (limits included) is well adapted.

The term "about", when referring to a measurable value, is meant to encompass variations of ±3% from the specified value, as such variations are appropriate to perform the disclosed methods.

As indicated above, it is advisable to pre-heat the composition arising from the first container before performing the heating step within the pipe. This is performed in a chamber out of the first container or in a pipe that conducts the composition from the first container to the pipe where it is heated at the desired temperature for the desired amount of time. The pre-heat temperature may be 20°C or 30°C less than the targeted heating temperature. Pre-heating between 75°C and 90°C (such as at 80°C) is well adapted.

In order to obtain the desired temperature within the pipe, and especially when heating by heat-exchange is performed, it is heated at a temperature higher than such desired temperature (generally about 5°C higher) as this takes into account the temperature gradient from the outside of the pipe to the center of the pipe. Heating can be performed by any of the following methods:
Direct steam injection: injecting steam directly into the pipes to heat them. The steam is generated in a boiler and directed into the pipes, where it transfers its heat to the pipe walls, heating the product flowing through.

Indirect heating by heat exchange: double-walled pipes are used. The composition flows through the inner tube, while steam circulates in the space between the two walls. The heat from the steam transfers through the pipe wall, heating the product.

Electric heating: heating elements are built into the pipes to heat them, electricity being used to generate heat directly in the pipe walls.

Induction beam heating: This method uses electromagnetic induction to heat the pipes, by Joule effect, by use of an alternating electric current induced in the pipe walls with the aid of a magnetic field.

In some embodiments, the composition is circulated within the pipe with a flow rate comprised between 15 and 30 Uh.

The composition is generally cooled and delivered to a second container after flowing. In some embodiments, the soluble fraction, that has been recovered by centrifugation and/or filtration after flowing and heating is delivered to the second container. Further purification or concentration of casein (for instance by ways of evaporation) and/or of quantification of the casein in the soluble fraction can then be performed.

As indicated above, a specific embodiment encompasses the situation when the starting composition (composition containing the casein and other proteins) has been obtained from a bacterial culture. In such embodiment, the bacteria have been transformed with one or more nucleic acid coding for more than one casein.

In particular, in one embodiment, the bacteria have been transformed with one or more nucleic acid coding for a beta casein.

In particular, in one embodiment, the bacteria have been transformed with one or more nucleic acid coding for an alpha-S1 casein.

In particular, in one embodiment, the bacteria have been transformed with one or more nucleic acid coding for an alpha-S2 casein.

In particular, in one embodiment, the bacteria have been transformed with one or more nucleic acid coding for both a beta and an alpha-S1 casein.

In particular, in one embodiment, the bacteria have been transformed with one or more nucleic acid coding for a beta an alpha-S1 casein and an alpha-S2 casein, or a combination of two out of them.

It is to be noted that, when multiple proteins are produced, the bacteria can be transformed with different nucleic acids (each one coding for a different protein) or with a unique nucleic acid (which contains the elements allowing production of the various proteins). Such methods of transforming microorganisms and bacteria in particular, for production of one or of multiple proteins are known in the art.

The nucleic acids are such that they contain all elements allowing production of the casein that they encode (such as promoters, enhancers, terminators and the like). The person skilled in the art knows all these elements and is able to select the ones that are the most appropriate. The person skilled in the art can also select the microorganisms to use to produce the caseins.

In particular, prokaryotic hosts suitable for expressing caseins include *E. coli, Bacillus subtilis, Salmonella typhimurium, Lactococcus lactis* and various species within the genera *Lactococcus, Pseudomonas, Streptomyces,* and *Staphylococcus.*

Eukaryotic host suitable for expressing caseins include fungi, such as *Saccharomyces cerevisae, Kluyveromyces lactis, Pichia pastoris, or Trichoderma reesei*).

Plant cells may also be used for producing protein, either in cell culture or in whole plants.

Further steps can be added such as addition of chemical resins, activated charcoal, membrane filtration (notably ultrafiltration, nanofiltration, or reverse osmosis), centrifugation, chromatography or precipitation of casein, resulting in a process comprising
i) providing a microorganism composition (preferably bacterial composition), wherein the microorganism composition comprises microorganisms (preferably bacteria) that have been transformed with at least one nucleic acid coding for a casein, and wherein the microorganisms have been cultured in culture conditions so as to express and produce casein, wherein the pH of the composition is equal or above 6.5 and preferably below 9
ii) optionally washing the microorganism composition to remove the culture medium
iii) optionally lysing the microorganism composition to obtain a liquid composition wherein the liquid composition is in a first container
iv) flowing the liquid composition out of the first container within device comprising a pipe and wherein the liquid composition is heated at a temperature equal or above 75°C during flowing within the pipe, for at most 5 minutes
v) recovering the soluble fraction out of the pipe
vi) further processing said soluble fraction by adding at least one step chosen among addition of activated charcoal, membrane filtration, chromatography or casein precipitation
thereby obtaining a casein composition.

In this embodiment, heating during flowing in iv) also induces lysis of the microorganisms, so that a microbial extract is obtained, and casein is enriched and/or other proteins are reduced in this extract.

In addition, the heating process can be used independently of cell lysis, in a process comprising:
i) providing, in a first container, a microbial extract, wherein the microbial extract results from the lysis of a microbial culture comprising micro-organisms that have been transformed with a nucleic acid coding for the casein, and cultured so as to express and produce casein
ii) flowing the microbial extract out of the first container within a device comprising a pipe and wherein the liquid composition is heated at a temperature equal or above 75°C during at least part of the flowing within the pipe for at most 5 minutes,
iii) recovering the soluble fraction out of the pipe
iv) further processing said soluble fraction by adding at least one step chosen among addition of chemical resins, activated charcoal, membrane filtration, centrifugation, chromatography or casein precipitation
thereby obtaining a casein composition.

The soluble fraction recovered in iii) can be further processed for increasing purity of casein.

Activated charcoal can be added to the soluble fraction and stirring is performed. Activated charcoal can be used to adsorb impurities (in particular organic impurities or chlorine) present in the soluble fraction, and that were not removed when recovering the soluble fraction in iii). Stirring with activated charcoal is preferably done, and the activated charcoal is removed before another method (such as the ones disclosed below) is performed on the soluble fraction.

Chromatography methods are widely used for the purification methods, and include notably affinity chromatography, ion exchange chromatography, hydrophobic interaction chromatography and others.

Membrane filtration, including notably ultrafiltration and nanofiltration is commonly used in protein purification as well (Saxena et al. (2009) Membrane-based techniques for the separation and purification of proteins: An overview. Advances in colloids and Interface Science Volume 145, pages 1-22), and membrane filtration techniques are widely used in the dairy industry. Interestingly, they can be used to separate the different caseins from each other (see above).

Finally, the specific properties of caseins (Post et al. (2012) Effect of temperature and pH on the solubility of caseins: Environmental influences on the dissociation of caseins. J. Dairy Sci. Volume 95 : pages 1603-1616), and notably, their propensity to precipitate in acidic conditions can be used for further purification.

In a preferred embodiment, further processing said soluble fraction is done by precipitation of caseins in acidic conditions. After precipitation, caseins can be resuspended, and using an appropriate basic buffer, they can also be resolubilized (Post et al. (2012) Effect of temperature and pH on the solubility of caseins: Environmental influences on the dissociation of caseins. J. Dairy Sci. Volume 95 : pages 1603-1616). In a preferred embodiment, further processing of said soluble fraction is done by precipitation of caseins at pH ranging from pH=4 to pH=5. In a more preferred embodiment, precipitation of caseins is conducted at pH of about 4.6. Various acids can be used including lactic acid, hydrochloric acid, and sulfuric acid.

Casein can then be recovered by methods such as centrifugation, or tangential filtration. A washing step can be added, with water adjusted at the pH used for precipitation. Separation of the washed casein can be made using the method as cited above.

Precipitation in acidic conditions is particularly interesting when the casein is isolated from a culture of microorganisms, as it makes it possible to remove or degrade any nucleic acid that may be present in the casein composition. Presence of DNA or RNA of the microorganisms may prove to be detrimental (at least from a regulatory point of view) when the casein is to be used for obtaining edible compositions intended to be ingested by human beings (such as cheese substitutes). pH is in the range of, and at about 4.6, and temperature ranging from 80°C to 140°C and times may vary between a few minutes and 2 hours.

Alternatively, recombinant DNA can be eliminated using nucleases such as DNAses that can be eliminated by heating.

When using microorganisms such as Gram- bacteria, one also could add other steps for elimination of lipopolysaccharides (LPS), and treatment at high temperature in acidic or basic conditions, the use of activated charcoal at high temperature, or filtration methods (10-20kDa separation, or various extraction meythods could be used).

pH can then be neutralized to obtain caseinates. For this, various basic coumpounds can be added such as liquid or gaseous ammonia, sodium hydroxide, potassium hydroxide or calcium hydroxide. Targeted pH can range between 6.5 and 9/. The kinetic of pH adjustment can continuous, or following a series of plateau, in order to achieve a stable target pH. before drying, caseins can be cocenctrated by filtration or evaporation. Caseins can then be dessicated by spray drying, flash drying, or other methods known in the field.

In a preferred embodiment, pH is adjusted using sodium hydroxide. In another embodiment, pH is adjusted using potassium hydroxide. In another embodiment, pH is adjusted using calcium hydroxide.

In a specific embodiment, the casein composition is desiccated. When obtained from a bacterial culture, such composition shall contain about 15-30% casein (w/w) and carbohydrates in the desiccated composition.

It is preferred when a little water is left, to favor future rehydration. Consequently, desiccation is to be understood as reducing the amount of water. In some embodiments, the amount of water is about 50% or less (w/w).

The insoluble fraction, containing most of the biomass produced during fermentation, can be separately treated and valorized. Such biomass can be used in feed or food applications, after using the same treatment as for caseins to remove recombinant DNA: pH is in the range of 4.6, with temperature ranging from 80°C to 140°C, and time may vary between a few minutes and 2 hours; alternatively, recombinant DNA can be eliminated using nucleases such as DNAses that can be eliminated by heating.

In specific embodiments, the invention relates to a method for the production of a casein composition comprising:
i) providing, in a first container, a bacterial composition, wherein the bacterial composition comprises bacteria that have been transformed with at least one nucleic acid coding for a casein, and the bacteria have been cultured so as to express and produce casein,
ii) flowing the bacterial composition out of the first container within a device comprising a pipe and heating the composition during flowing through the pipe, wherein heating is performed at a temperature equal or above 75°C, for at most 5 minutes
iii) recovering the soluble fraction from the heated cell composition of ii),
iv) optionally and preferably further processing said soluble fraction by adding at least one step chosen among addition of activated charcoal, membrane filtration, chromatography or casein precipitation
thereby obtaining a casein composition (in the soluble fraction).

When the bacteria of the bacterial composition are not lysed, heating in ii) also induces lysis of the cells, which produces a bacterial extract.

In other embodiments, the invention relates to a method for the production of a casein composition comprising:
i) providing, in a first container, a bacterial composition, wherein the bacterial composition comprises bacteria that have been transformed with at least one nucleic acid coding for a casein, and the bacteria have been cultured so as to express and produce casein,
ii) flowing the bacterial composition out of the first container within a device comprising a pipe and heating the composition during flowing through the pipe, wherein heating is performed at a temperature equal or above 75°C, for at most 5 minutes
iii) recovering the soluble fraction from the heated cell composition of ii),
iv) further processing said soluble fraction by precipitation of casein in acidic conditions
thereby obtaining a casein composition (in the precipitate).

In this embodiment, when the bacteria of the bacterial composition are not lysed, heating in ii) shall also induce lysis of the bacteria, thereby producing a bacterial extract.

In other embodiments, the invention relates to a method for the production of a casein composition comprising:
i) providing, in a first container, a bacterial composition, wherein the bacterial composition comprises bacteria that have been transformed with at least one nucleic acid coding for a casein, and the bacteria have been cultured so as to express and produce casein,
ii) flowing the bacterial composition out of the first container within a device comprising a pipe and heating the composition during flowing in the pipe, wherein heating is performed at a temperature equal or above 75°C, for at most 5 minutes
iii) recovering the soluble fraction from the heated cell composition of ii),
iv) further processing said soluble fraction by precipitation of casein by heating at 90°C at pH=4
thereby obtaining a casein composition (in the precipitate).

In a preferred embodiment, heating the bacterial composition induces lysis of the cells.

The methods herein disclosed thus make it possible to obtain a casein composition. Such a casein composition susceptible to be obtained (or obtained) by a method herein disclosed is a further subject of the invention. Such composition can be characterized as defined above.

In milk, the casein is in the micellar form found in milk, wherein micelles include alpha-S1, alpha-S2, beta and kappa caseins assembled in the same particules. In the context of the present invention, casein may not be assembled in the micellar form. However, this doesn't prevent ability to obtain a curd from the casein composition, by addition of an appropriate curdling agent.

Indeed, as developed in the examples, such casein composition can be used for producing cheese substitutes, and in particular animal-free cheese substitutes, *i.e.* cheese substitutes that don't contain any products of animal origin (in particular when the casein has been isolated from bacterial or yeast culture).

In summary, simple and short (less than 5 minutes, or preferably less than 2 minutes) heating of a composition containing casein and other proteins through a pipe makes it possible to enrich the composition in casein by reducing the amount of most of the other proteins in the soluble fraction. Heating also makes it possible to solubilize casein found in the insoluble fraction of bacterial cultures. The obtained casein, although not in the optimal condition for forming micelles, can appropriately curd upon the action of a curdling agent. Such findings were unexpected.

In particular, as shown in WO2022253816, curdling can be achieved with the recombinant caseins made in bacteria, and in the absence of kappa caseins, even though they don't possess post-translation modifications (such as phosphorylations) and the fact that kappa caseins are known to play an important role in the formation of dairy casein micelles.

The compositions herein disclosed, and obtained or susceptible to be obtained by the methods disclosed above, thus can be used in a method for obtaining a curd, comprising
i) providing a casein composition herein disclosed,
ii) mixing said casein composition with at least one other ingredient comprising at least one component selected from the group consisting of water, calcium, lipids, and carbohydrate, so as to obtain a liquid pre-curd composition (LpCC)
iii) Adding at least one curdling agent to the liquid composition so as to obtain a curd.

Such embodiment is disclosed notably in WO2022058573 and WO2022253816.
As indicated, said curdling agent is preferably not rennet, but is an acidifying agent, notably a lactic bacteria, or lactic, citric or acetic acid. The casein composition may contain only alpha-S1 casein (as the casein protein), or only alpha-S2 casein (as the casein protein), or only beta casein (as the casein protein), or only alpha-S1 and beta casein (as the casein proteins), or only alpha-S2 and beta casein (as the casein proteins), or only alpha-S1 and alpha-S2 casein (as the casein proteins), or alpha-S1, alpha-S2 and beta casein.

The curd may be processed to obtain an edible composition, notably a cheese substitute, as disclosed in WO2022058573 and WO2022253816. These two documents are herein incorporated by reference, in particular the parts describing the obtention of a LpCC or of an edible composition, starting from a composition containing caseins, notably produced in bacteria and/or without kappa casein, in particular parts pertaining to obtaining a cheese substitute having the essential features of fresh cheese and a cheese substitute having the essential features of soft cheese or semi-soft cheese. The parts of these documents and in particular of WO2022253816, pertaining to calcium and other salts, lipids, emulsifying and gelling agents, carbohydrates, vitamins, curdling agents, ferments, moisture, and processing of the curd are also incorporated by reference.

It is reminded that a gelling agent (in particular agar-agar) can be added in with the other ingredients before curdling or after curdling.

It is preferred when the other ingredients added in b) are all of non-animal origin.

The ingredients added in ii) comprise
i. Optionally proteins other than caseins
ii. Lipids
iii. Water, and
iv. Carbohydrates; in preferred embodiments, the carbohydrates do not comprise lactose, in order for the edible composition to be more acceptable to lactose-intolerant customers.

The choice and amount of the ingredients is adjusted so that the composition of the LpCC is adapted to the eventual composition desired for the edible composition, taking into account the loss of water due to curdling and aging (which can be modulated by one of skill in the art by modifying the duration and conditions of maturation).

The invention also relates to a process comprising:
i) Providing, in a first container, a bacterial composition, wherein the bacterial composition comprises bacteria that have been transformed with at least one nucleic acid coding for the casein (preferably not kappa casein), and the bacteria have been cultured so as to express and produce casein
ii) flowing the bacterial composition out of the first container within a device comprising a pipe and heating the composition during flowing within the pipe, wherein heating is performed at a temperature equal or above 75°C, for at most 5 minutes
iii) recovering the soluble fraction from the heated cell composition of ii)
iv) optionally and preferably further processing said soluble fraction by adding at least one step chosen among addition of activated charcoal, membrane filtration, chromatography or casein precipitation, thereby obtaining a casein composition
v) Mixing said casein composition with at least one other ingredient comprising at least one component selected from the group consisting of water, calcium, lipids, and carbohydrate, so as to obtain a liquid pre-curd composition (LpCC)
vi) Adding at least one curdling agent to the liquid composition so as to obtain a curd.

### DESCRIPTION OF THE FIGURES

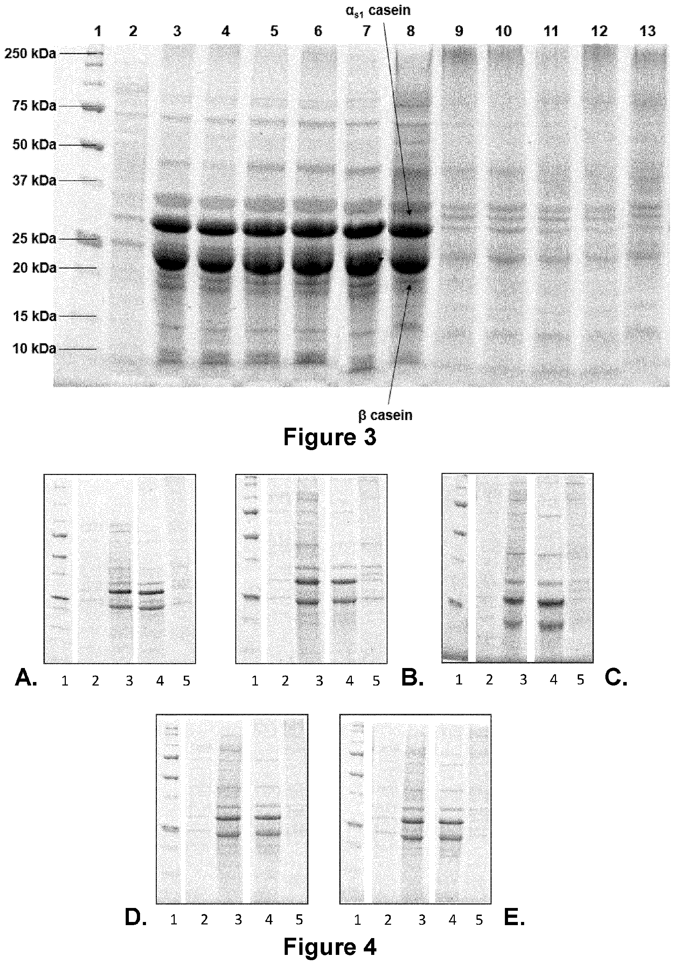

**Figure** 1: Structure of plasmid expressing beta and alpha-S1 caseins.
**Figure 2****:** Monitoring of the supply pressure and of the temperature's product during the heating step and at the exit of the pasteurization chamber (tubular heat exchanger). Plain line: heating temperature of the product. Small dotted line: product's temperature at the exit of the pasteurization chamber. Large dotted line: supply pressure.
   A. Trial 1: Holding time : 110°C - Heating time: 75 seconds
   B. Trial 2. Holding time : 110°C - Heating time: 30 seconds
   C. Trial 3. Holding time : 120°C - Heating time : 30 seconds
   D. Trial 4 : Holding time : 140°C - Heating time : 30 seconds
   E. Trial 5 : Holding time : 140°C - Heating time : 2 seconds
**Figure 3****:** SDS-PAGE analysis of the αₛ₁ and β caseins in the lysate, before and after thermal treatment, in soluble and insoluble fractions. **Lane 1:** Size marker, Precision Plus Protein^{™} Unstained Standards (Bio-Rad, 1610363EDU) - **Lane 2:** Cell suspension before thermal treatment, soluble fraction) - **Lane 3:** Lysate after thermal treatment (Trial 1), soluble fraction - **Lane 4:** Lysate after thermal treatment (Trial 2), soluble fraction - **Lane 5:** Lysate after thermal treatment (Trial 3), soluble fraction - **Lane 6:** Lysate after thermal treatment (Trial 4), soluble fraction - **Lane 7:** Lysate after thermal treatment (Trial 5), soluble fraction - **Lane 8:** Cell suspension before thermal treatment, insoluble fraction - **Lane 9:** Lysate after thermal treatment (Trial 1), insoluble fraction - **Lane 10:** Lysate after thermal treatment (Trial 2), insoluble fraction - **Lane 11:** Lysate after thermal treatment (Trial 3), insoluble fraction - **Lane 12:** Lysate after thermal treatment (Trial 4), insoluble fraction - **Lane 13:** Lysate after thermal treatment (Trial 5), insoluble fraction.
**Figure 4****:** SDS-PAGE analysis of the αₛ₁ and β caseins in the lysate, before and after thermal treatment, in soluble and insoluble fractions. Analysis of 3-fold diluted samples. For each panel, **Lane 1:** Size marker, Precision Plus Protein^{™} Unstained Standards (Bio-Rad, 1610363EDU) - **Lane 2:** Cell suspension before thermal treatment, soluble fraction - **Lane 3:** Cell suspension before thermal treatment, insoluble fraction - **Lane 4:** Lysate after thermal treatment, soluble fraction - **Lane 5:** Lysate after thermal treatment, insoluble fraction. Panel A: 110°C, 75 seconds; B: 110°C, 30 seconds; C: 120°C, 30 seconds; D: 140°C, 30 seconds; E: 140°C, 2 seconds.

### EXAMPLES

### Example 1: Production of alpha-S and beta caseins

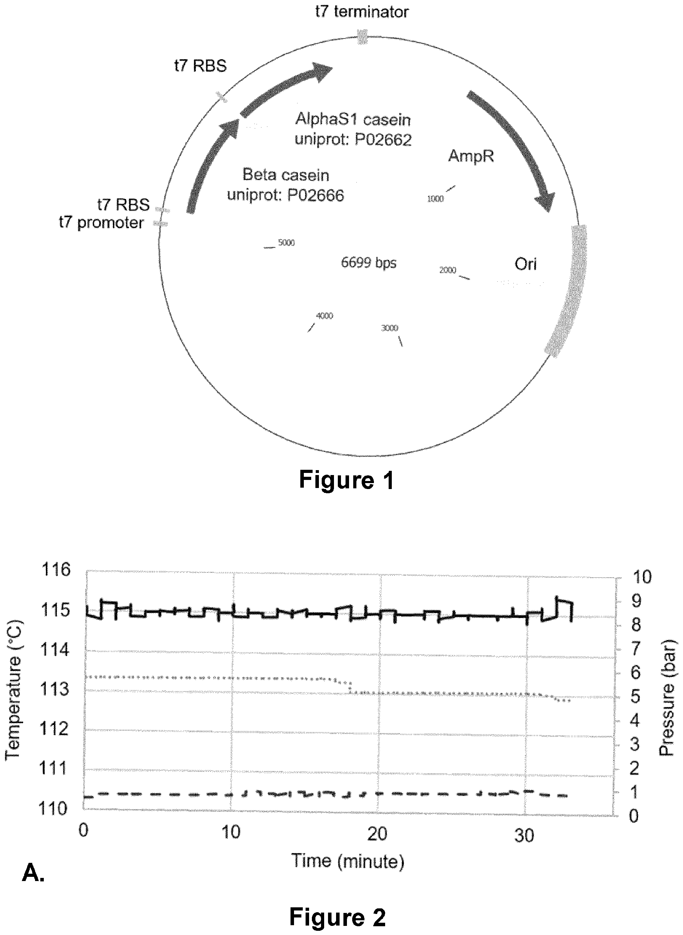

Synthetic genes coding for alpha-S1 and beta casein (related to natural genes P02662, and P02666, respectively), were modified, in order to remove the signal peptide, and the sequence of the new synthetic open reading frames (SEQ ID NO: 2 and SEQ ID NO: 4) are shown in Table 1, last column. They were cloned into pET25b+ (Figure 1) and the resulting plasmid was transformed into BL21(DE3) strains (Novagene). Individual transformed clones were isolated, and for each synthetic gene, one clone was used to inoculate LB medium.

**Table 1: Sequences of natural caseins (precursors) and of the related recombinant proteins. In natural caseins, signal peptides are indicated in bold.**

| Name and Uniprot reference of protein precursor | sequence of protein precursor | sequence of recombinant proteins |
|---|---|---|
| Alpha-S1 casein P02662 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EQLLRLKKYKVPQLEIVPNSAE ERLHSMKEGIHAQQKEPMIGV NQELAYFYPELFRQFYQLDAY PSGAWYYVPLGTQYTDAPSFS DIPNPIGSENSEKTTMPLW (SEQ ID NO: 1) | MRPKHPIKHQGLPQEVLNENLLR FFVAPFPEVFGKEKVNELSKDIG SESTEDQAMEDIKQMEAESISSS EEIVPNSVEQKHIQKEDVPSERY LGYLEQLLRLKKYKVPQLEIVPNS AEERLHSMKEGIHAQQKEPMIGV NQELAYFYPELFRQFYQLDAYPS GAWYYVPLGTQYTDAPSFSDIPN PIGSENSEKTTMPLW (SEQ ID NO: 2) |
| Beta casein P02666 | MKVLILACLVALALARELEELN VPGEIVESLSSSEESITRINKKIE | MRELEELNVPGEIVESLSSSEESI TRINKKIEKFQSEEQQQTEDELQ |
| | KFQSEEQQQTEDELQDKIHPF AQTQSLVYPFPGPIPNSLPQNI PPLTQTPVVVPPFLQPEVMGV SKVKEAMAPKHKEMPFPKYPV EPFTESQSLTLTDVENLHLPLP LLQSWMHQPHQPLPPTVMFPP QSVLSLSQSKVLPVPQKAVPY PQRDMPIQAFLLYQEPVLGPV RGPFPIIV (SEQ ID NO: 3) | DKIHPFAQTQSLVYPFPGPIPNSL PQNIPPLTQTPVVVPPFLQPEVM GVSKVKEAMAPKHKEMPFPKYP VEPFTESQSLTLTDVENLHLPLPL LQSWMHQPHQPLPPTVMFPPQ SVLSLSQSKVLPVPQKAVPYPQR DMPIQAFLLYQEPVLGPVRGPFP IIV (SEQ ID NO: 4) |

In order to produce a batch of alpha-S1 and beta caseins, the resulting strain was cultivated as follow:
Five concentrated stocks of the strain of each 1 mL, stored in 10 % DMSO at - 80°C, were thawed and used to inoculate five precultures of a volume of 1 L each, respectively (0.1 % *v*/*v* inoculation rate), in a revivification medium Y15 composed of yeast extract (15 g/L) and NaCl (5 g/L), and supplemented with 100 µg/mL of ampicillin. The five precultures were grown in 4 L or 5 L erlenmeyers at 30°C, for 9 hours, at 170 rpm (orbital diameter: 25 mm). After 9 hours, optical densities (60nm) in the range of 3 were achieved.

2.4 L of the first preculture were used to inoculate a volume of 120 L (2% *v*/*v* inoculation rate), in an optimized culture media supplemented with 100 µg/mL of ampicillin and with 15 g/L of glucose. Growth parameters (oxygen pressure, stirring, pressure, aeration rate and pH regulation) were optimized to achieve an optical density (600nm) in the range of 15.

90 L of this culture were used to inoculate a fermentor with an initial volume of 1500 L (6 % v/v inoculation rate), in an optimized culture media, supplemented with 100 µg/mL of ampicillin. Growth parameters (glucose feed, oxygen pressure, stirring, pressure, aeration rate and pH regulation) were optimized to achieve an optical density (600nm) in the range of 90. IPTG was added at a final concentration of 0,2mM when optical density reached 20. This resulted in around 2 tons of culture broth with 3,4% of dry biomass.

### Example 2: Thermal bacterial lysis by ultra-high temperature processing (UHT)

A protocol was set for extraction and solubilization of αₛ₁ and β caseins from cells at ultra-high temperature.

### Biomass concentration and washing

The culture medium obtained as described in Example 1 was concentrated using a self-cleaning disk-stack centrifuge (SSE20, GEA, centrifugal force : 20000 G, bowl rotation speed : 12000 rpm, bowl filling : 80% of the capacity, feed rate : 500 Uh, back pressure : 5 bars, temperature < 20 °C, settling time : 54 seconds for the first solid/liquid separation (biomass concentration) and 40 seconds for the three washing steps of the biomass). 298 kgs of concentrated cells were collected, while 1670 kgs of fermentation medium was discarded.

The concentrated biomass was then washed three times with osmosis water, using the same centrifuge. The concentrated biomass was mixed with water, until obtaining a homogeneous suspension (11.17 % of dry mass). The suspension was then separated by centrifugation, and the first fraction of washing water (800.4 kg) was eliminated. This washing procedure was repeated twice; to finally obtain a pellet of concentrated cells of 239.1kg. The color of the supernatant was less and less yellow, until obtaining a slightly colored yellow washing water at third wash.

### Thermal bacterial lysis

Washed concentrated biomass was then resuspended in 4,2 volumes of osmosis water, until having a homogeneous suspension. 60 L of this resulting suspension were stored during four days at +4°C, without any stirring. After four days, the pH of the suspension was 6.654 ± 0.057. The suspension was then divided in four fractions of 10 kgs, and one fraction of 8.15 kgs. The pH of each suspension was adjusted at 8.5, using a 2 M solution of sodium hydroxide (NaOH). pH and temperature of the different fractions collected before and after the pH adjustment are described in **Table 2.**

**Table 2: pH and temperature of the different fractions before and after pH adjustment to 8.5**

| **Trial** | **Product** | **pH** | **Temperature** |
|---|---|---|---|
| | Washed biomass | 6.620 | 7°C |
| 1 | After pH adjustment | 8.501 | 20°C |
| 2 | Washed biomass | 6.700 | 10°C |
| | After pH adjustment | 8.500 | 22°C |
| 3 | Washed biomass | 6.700 | 18°C |
| | After pH adjustment | 8.509 | 34°C |
| 4 | Washed biomass | 6.680 | 15.6°C |
| | After pH adjustment | 8.481 | 21°C |
| 5 | Washed biomass | 6.570 | 12°C |
| | After pH adjustment | 8.498 | 22°C |

The five fractions were then heated by monitoring a holding time at a given temperature in a pasteurization chamber ), using a UHT Pasteurizer (Pasteurizer, OMVE, HTST/UHT System, HT220-DSI, initial temperature of the product : 20°C, configuration : tubular heat exchanger, flow rate : 16.66-27.2 Uh, holding time : 2-75 seconds, pre-heating temperature : 80°C, heating temperature : 113-145°C, temperature in the pasteurization chamber: 110-140°C, pre-cooling temperature : 80°C, cooling temperature : 40°C, pressure : 5 bars).

Five different conditions were tested on the five bacterial suspensions, in order to determine an optimal and fast thermal treatment, allowing efficient bacterial lysis, *E.coli* proteins precipitation and caseins solubilization. In each case, the applied flow rate, the holding time, the pre-heating and heating temperatures, and the pre-cooling and cooling temperatures are described in **Table 3.** The different set heating temperatures were slightly higher than the temperatures applied in the pasteurization chamber, to ensure that those last temperatures would be reached.

| | |
|---|---|
| **Trial 1** : | temperature = 110 °C, holding time = 75 seconds |
| **Trial 2** : | temperature = 110°C, holding time = 30 seconds |
| **Trial 3** : | temperature = 120 °C, holding time = 30 seconds |
| **Trial 4 :** | temperature = 140 °C, holding time = 30 seconds |
| **Trial 5** : | temperature = 140 °C, holding time = 2 seconds, at micro-pilot scale |

The general conditions are described in detail in Table 3. All of these different conditions allow a total deactivation of the bacterial *E.coli* strain used in fermentation.

**Table 3: Experimental conditions of the five different trials of thermal treatments**

| **Trial** | **Parameters** |
|---|---|
| 1 | Product: 10 kgs |
| | Initial temperature of the product: 20°C |
| | Configuration : tubular heat exchanger |
| | Flow rate : 16.66 L/h |
| | Holding time: **75 seconds** |
| | Pre-heating temperature : 80°C |
| | Heating temperature : 115°C |
| | Temperature in the pasteurization chamber: **110°C** |
| | Pre-cooling temperature : 80°C |
| | Cooling temperature : 40°C |
| 2 | Product: 10 kgs |
| | Initial temperature of the product: 20°C |
| | Configuration : tubular heat exchanger |
| | Flow rate : 27.2 L/h |
| | Holding time: **30 seconds** |
| | Pre-heating temperature : 80°C |
| | Heating temperature : 113°C |
| | Temperature in the pasteurization chamber: **110°C** |
| | Pre-cooling temperature : 80°C |
| | Cooling temperature : 40°C |
| 3 | Product: 10 kgs |
| | Initial temperature of the product: 20°C |
| | Configuration : tubular heat exchanger |
| | Flow rate : 27.2 L/h |
| | Holding time: **30 seconds** |
| | Pre-heating temperature : 80°C |
| | Heating temperature : 124°C |
| | Temperature in the pasteurization chamber: **120°C** |
| | Pre-cooling temperature : 80°C |
| | Cooling temperature : 40°C |
| 4 | Product: 10 kgs |
| | Initial temperature of the product: 20°C |
| | Configuration : tubular heat exchanger |
| | Flow rate : 27.2 L/h |
| | Holding time: **30 seconds** |
| | Pre-heating temperature : 80°C |
| | Heating temperature : 145°C |
| | Temperature in the pasteurization chamber: **140°C** |
| | Pre-cooling temperature : 80°C |
| | Cooling temperature : 40°C |
| 5 | Product: 8.5 kgs |
| | Initial temperature of the product: 20°C |
| | Configuration : tubular heat exchanger |
| | Flow rate : 20 L/h |
| | Holding time: **2 seconds** |
| | Pre-heating temperature : 80°C |
| | Heating temperature : 142°C |
| | Temperature in the pasteurization chamber: **140°C** |
| | Pre-cooling temperature : 80°C |
| | Cooling temperature : 40°C |

Product: amount of treated sample. Initial temperature of the product: initial temperature of the product to be treated. Configuration: tubular chamber (Pasteurization chamber) preceded by 2 heat exchangers (one heat exchanger where the outlet of the treatment heat up the product to be treated - one heat exchanger to complete the heat up prior the tubular chamber) and followed by 1 heat exchanger that cool down the treated product preliminary cool down. Flow rate: Flow rate of the product through the pasteurization chamber. Holding time: Time spent by the product in the pasteurization chamber. Pre-heating temperature: Temperature reached by the product at the outlet of the first heat exchanger. Heating temperature: Maximum temperature reached by the product at the inlet of the chamber. Temperature in the pasteurization chamber: Temperature of the product in the tubular chamber. Pre-cooling temperature: Temperature reached by the treated product at the coolest outlet of the first heat exchanger. Cooling temperature: Temperature of the treated product reached at the outlet of the final cooling heat exchanger.

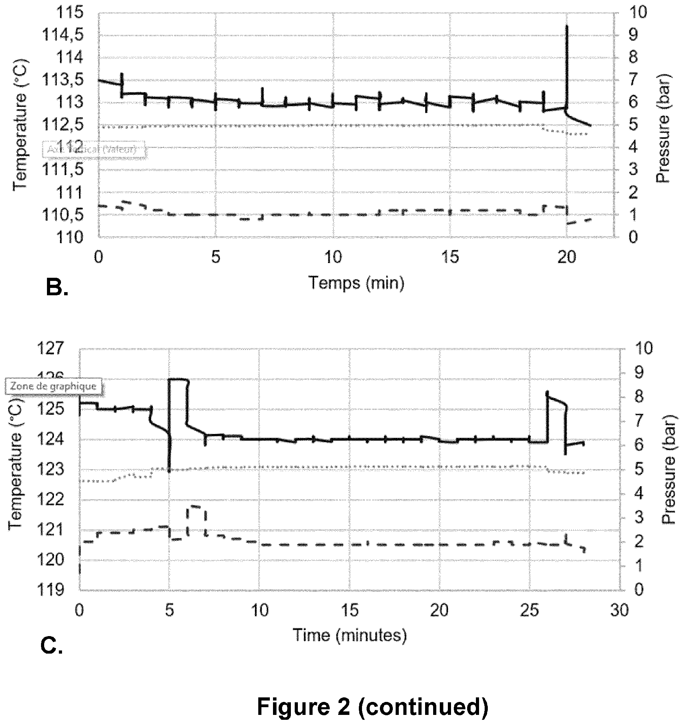

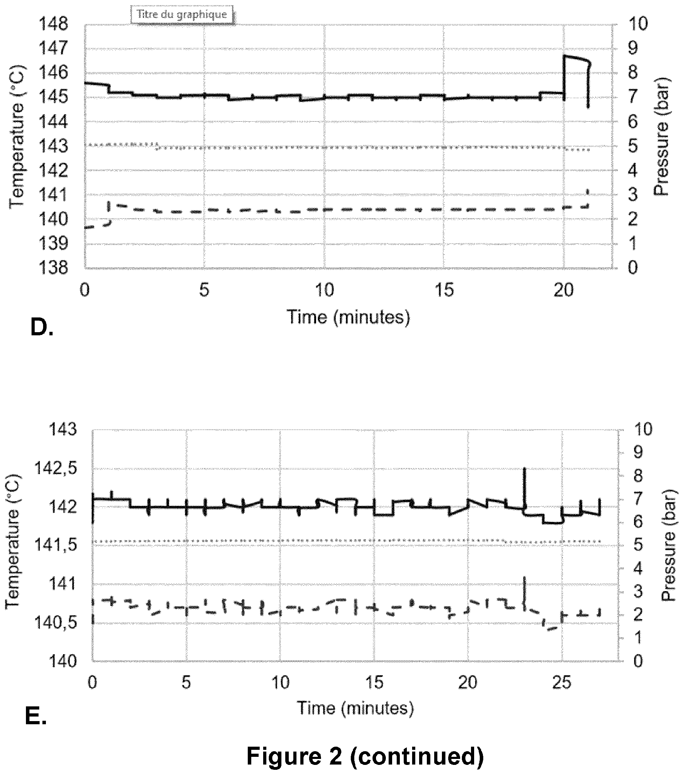

In each case, the temperature of the product was controlled during the heating step and at the exit of the pasteurization chamber, while the supply pressure was measured during all the process. The monitoring of these parameters is presented in Figures 2 to 6.

For trial 1 to trial 5, the weight of each fraction was measured, before and after the heating step, as well as the pH and the temperature. Moreover, the quantity of 2 M NaOH solution added to adjust the pH to 8.5 was also determined Table 4).

**Table 4: Monitoring of the pH, the temperature, and the weight of each fraction collected in trial 1 to trial 5, as well as the weight of 2 M NaOH solution added.**

| **Trial** | **Product** | **pH** | **Temperature** | **Weight [kg]** |
|---|---|---|---|---|
| 1 | Washed biomass | 6.620 | 7°C | 10.00 |
| | After pH adjustment | 8.501 | 20°C | - |
| | 2 M NaOH solution added | - | - | 27.30 |
| | After thermal treatment | 7.640 | 32°C | 9.165 |
| 2 | Washed biomass | 6.700 | 10°C | 10.00 |
| | After pH adjustment | 8.500 | 22°C | - |
| | 2 M NaOH solution added | - | - | 25.80 |
| | After thermal treatment | 7.593 | 30°C | 9.110 |
| 3 | Washed biomass | 6.700 | 18°C | 10.00 |
| | After pH adjustment | 8.509 | 19°C | - |
| | 2 M NaOH solution added | - | - | 25.60 |
| | After thermal treatment | 7.640 | 34°C | 9.030 |
| | Washed biomass | 6.680 | 15.6°C | 10.00 |
| | After pH adjustment | 8.481 | 21°C | - |
| 4 | 2 M NaOH solution added | - | - | 27.74 |
| | After thermal treatment | 7.790 | 32°C | 8.900 |
| 5 | Washed biomass | 6.570 | 12°C | 8.15 |
| | After pH adjustment | 8.498 | 22°C | - |
| | 2 M NaOH solution added | - | - | 22.18 |
| | After thermal treatment | 7.650 | 34°C | 7.115 |

After the different applied thermal treatments, pH of each fraction decreased, from 7.790 to 7.593, *i.e.* a decrease of 0.71 to 0.90 pH unit. From 10 kgs of product, between 8.9 kgs to 9.165 kgs were recovered, in trial 1 to trial 4. For the last trial, a fraction of 7.115 kg was obtained from an initial weight of 8.15 kgs.

In a view to investigate the efficiency of these five different heat treatments, samples were collected before and after each trial and analyzed by sodium dodecyl sulfate-polyacrylamide gel electrophoresis (SDS-PAGE) on a polyacrylamide gel (4-20% Criterion^{™} TGX Stain-Free Protein Gel, Bio-Rad, 5678093) with Precision Plus Protein^{™} Unstained Standards (Bio-Rad, 1610363EDU) as ladder.

For each trial, 1 mL of bacterial suspension was taken before and after the applied heat treatment, and centrifuged (13000 rpm, 5 minutes, RT), to separate the soluble and insoluble fractions. The supernatant was separated from the pellet, and mixed in a *1:1* ratio with the 2X sample buffer, composed of 0,1 M Tris-HCl, *4% v:v* SDS, 0,2% *w:v* bromophenol blue and 20 % *v:v* glycerol. The cell pellet was mixed with 1 mL of a 2% *v:v* SDS solution until obtaining a homogeneous suspension, and mixed in the same ratio with the 2X sample buffer previously described. Each resulting solution was heated at 95°C for 5 minutes and loaded in the wells of a polyacrylamide gel, using a running buffer composed of Tris-HCl (25 mM), glycine (250 mM) and SDS (0,1% *v:v*). The gel was then visualized under UV light, in a Gel-Doc^{™} EZ Imager (Bio-Rad). Diluted samples (3-fold) were also used for better quantification. The ratio of casein content vs total protein content was determined *via* the analysis of each SDS PAGE gels using the software Imagelab, by comparing bands' intensity of caseins and bands' intensity of other proteins from *E.coli.*

Results are summarized in Figures 3 and 4, with undiluted (Figure 3) and diluted (Figure 4) samples. In all tested conditions for pasteurization, *E.coli* cells were efficiently lysed and a large majority of αₛ₁ and β caseins were solubilized: total amounts of caseins amounts were very similar before and after pasteurization, but these caseins were essentially in the insoluble fraction before pasteurization (Figure 3, lane 8) and in the soluble fraction after pasteurization (Figure 3, lanes 3-7), indicating the process yield was very high (at least >80%).

The proportion of casein vs. total protein in the soluble fraction of the lysate (Figure 4, lanes 4 in each panel) was significantly the same in the five trials, ranging from ranging from 75 % to 77 %. This ratio appeared to be significantly higher than in the insoluble faction of cells before lysis (Figure 4, lanes 3 in each panel), which displayed many additional proteins bands, as can be seen on Figure 4 (lanes 3 in each panel). This ratio was a fortiori higher than in the cell composition (insoluble + soluble) before lysis, showing that the lysate soluble fraction was enriched in caseins as compared with the initial composition.

In addition, the average αₛ₁ casein/β casein ratio was also very similar between the five trials, ranging from 47% to 53%, similar to the initial composition in the cell before treatment, showing that in the condition we used, the was no bias regarding the purification of αₛ₁ casein vs. β casein.

## Claims

1. A method for obtaining a casein composition comprising:
i) providing a microorganism composition, wherein the microorganism composition comprises microorganisms that have been transformed with at least one nucleic acid coding for a casein, and wherein the microorganisms have been cultured in culture conditions so as to express and produce casein, wherein the pH of the composition is equal or above 6.5 and preferably below 9
ii) optionally washing the microorganism composition to remove the culture medium
iii) optionally lysing the microorganism composition to obtain a liquid composition wherein the liquid composition is in a first container
iv) flowing the liquid composition out of the first container through a device comprising a pipe and wherein the liquid composition is heated at a temperature equal or above 75°C during at least part of the flowing for at most 5 minutes,
v) recovering the soluble fraction out of the pipe
thereby obtaining a casein composition in the soluble fraction.

2. The method of claim 1, wherein the caseins represent at least 60 %, preferably at least 62 %, preferably at least 65 %, preferably at least 67 %, preferably at least 70 % of the total proteins of the soluble fractions

3. The method of claim 1 or 2, wherein the ratio of casein as compared to the total proteins is increased in the soluble fraction as compared to the ration in the microorganism composition.

4. The method of any one of claims 1 to 3, wherein heating is performed at a temperature equal or higher than 95°C.

5. The method of any one of claims 1 to 4, wherein heating is performed for at most 2 minutes.

6. The method of any one of claims 1 to 5, wherein heating is performed for at least 2 seconds.

7. The method of any one of claims 1 to 6, wherein heating is performed for at most 60 seconds.

8. The method of any one of claims 1 to 7, wherein the composition is circulated within the pipe at a flow rate with a comprised between 15 Uh and 30Uh.

9. The method of any one of claims 1 to 8, wherein the composition is delivered to a second container after flowing.

10. The method of any one of claims 1 to 8, wherein the soluble fraction is recovered by centrifugation and/or filtration after flowing and heating before being delivered to a second container.

11. The method of any one of claims 1 to 10, wherein the microorganisms in the microorganism composition are not lysed and heating in iv) lyses the microorganisms.

12. The method of any one of claims 1 to 11, wherein the microorganisms have been transformed with one or more nucleic acid coding for more than one casein.

13. The method of any one of claims 1 to 12, wherein the casein is selected from beta casein, alpha-S1 casein, alpha-S2 casein and mixture of these caseins.

14. The method of any one of claims 1 to 12, wherein the casein is selected from beta casein, alpha-S1 casein and mixture of these caseins.

15. The method of any one of claims 1 to 14, further comprising a step of performing a further purification of casein of the soluble fraction obtained in iii).

16. The method of claim 15, wherein the further step applied to the soluble fraction comprises at least one step selected in the group consisting of addition of activated charcoal, membrane filtration, chromatography and casein precipitation.

17. The method of claim 16, wherein the further step is acidic precipitation of casein.

18. The method of claim 17, wherein the further step of acidic precipitation of casein is conducted by heating at 90°C at pH=4.6.

19. The method of any one of claims 1 to 18, wherein the microorganism composition is a bacterial composition

20. The method of claim 19, wherein the bacteria are *E. coli.*

21. A casein composition susceptible to be obtained by a method according to any one of claims 1 or 20.
